(19) Europäisches Patentamt
European Patent Office
Office européen des brevets

(11) EP 0 818 438 A1

(12) **EUROPEAN PATENT APPLICATION**
published in accordance with Art. 158(3) EPC

(43) Date of publication:
**14.01.1998 Bulletin 1998/03**

(21) Application number: **96939945.0**

(22) Date of filing: **27.11.1996**

(51) Int. Cl.[6]: **C07C 229/36**, C07C 237/20, C12P 13/04, C12P 41/00

(86) International application number:
**PCT/ES96/00226**

(87) International publication number:
**WO 97/19907 (05.06.1997 Gazette 1997/24)**

(84) Designated Contracting States:
**AT BE CH DE DK ES FI FR GB GR IE IT LI LU MC NL PT SE**

(30) Priority: **27.11.1995 ES 9502328**

(71) Applicant:
**DERIVADOS DEL ETILO, S.A.**
**8007 Barcelona (ES)**

(72) Inventors:
- **ESTEBAN MORALES, Manuel**
  **E-04618 Villaricos (ES)**
- **PALLARES BAYO, Antonio**
  **E-04618 Villaricos (ES)**

(74) Representative:
**Carpintero Lopez, Francisco**
**HERRERO & ASOCIADOS, S.L.**
**Alcalá, 21**
**28014 Madrid (ES)**

# (54) CHIRAL DERIVATIVES OF HYDROXYPHENYLGLYCIN, AND THEIR USE IN THE SYNTHESIS OF PHARMACEUTICAL ACTIVE PRINCIPLES

(57) The chiral derivates of hydroxyphenylglycine, with the D configuration, have the general formula (I), where R is H, hydroxy, chloride, $-NH_2$, -NHW, where W is an alkyl group $C_1$ - $C_4$, or aryl, -OR', where R' is an alkyl group $C_1$ - $C_4$, and Z is H, $-CONH_2$ and $-C(CH_3)=CHCOOY$, where Y is a methyl or ethyl. One of these compounds, the D-(-)-2-(2'-hydroxyphenyl) glycine may be obtained using enzymatic means from the corresponding hydantoin while the others may be obtained chemically or enzymatically.

EP 0 818 438 A1

## Description

### FIELD OF THE INVENTION

The invention refers to new chiral derivates of hydroxyphenylglycine, and in particular to D-(-)-2-(2'-hydroxyphenyl) glycine and its derivates, its preparation and use in the manufacture of active pharmaceutical ingredients.

### BACKGROUND OF THE INVENTION

E.K. Harvill and R.M. Herbst [J. Org. Chem., 9, 21-30 (1944)] described the obtention of racemic 2-(2'-hydroxyphenyl) glycine, although they describe neither its pure entantiomers nor its chiral derivates.

The use of racemic mixtures in the synthesis of pharmaceutical active ingredients has, amongst other drawbacks, the disadvantage that in one of the stages of the process a resolution stage has to be performed to separate out the desired optical isomer as, in general, one of the said isomers may be inactive or not very active, while the other may present a high rate of activity and, on occasions, one of the isomers may give rise to undesirable side - effects. These resolution stages may be difficult and costly to carry out. Therefore, from a practical point of view, it is convenient to start from the suitable isomer in those processes which lead to the obtaining of the final chiral products with a specific configuration.

### DETAILED DESCRIPTION OF THE INVENTION

This invention provides new chiral derivates of hydroxyphenylglycine, with the D configuration, and general formula (I)

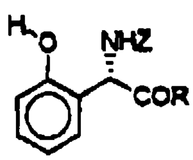

(I)

where

R is hydrogen, hydroxy, chloride, $-NH_2$, -NHW, where W is an alkyl group $C_1$ - $C_4$ or aryl, -OR', where R' is an alkyl group $C_1$ - $C_4$; and
Z is hydrogen, $-CONH_2$, and -C $(CH_3)$=CHOOY, where
Y is methyl or ethyl;

their salts, solvates or hemisolvates.

These compounds of formula (I) are useful in the production of pharmaceutical drugs, and in particular for β - lactamic antibiotics.

The term "alkyl group $C_1$ - $C_4$" refers to an alkyl radical having from 1 to 4 carbon atoms, in a lineal or branching chain.

The term "aryl group" refers to an aromatic radical having at least 6 carbon atoms.

The invention also covers the metallic salts of the compounds included within formula (I), and in particular the alkaline and alkali earth metallic salts, as well as acid and base addition salts, and in particular hydrochloride and the solvates or hemisolvates which may be obtained using organic solvents.

The preferred compounds of formula (I) are those in which:

Z is hydrogen and R is -OH.
Z is - C(CH3)=CHCOOY, where Y is methyl or ethyl, and R is $-O^-K^+$ or $O^-Na^+$;
Z is -H.Cl and R is -Cl, in the form of a hemisolvate with dioxane;
Z is H and R is $-NH_3$, or NHW, where W is a lineal alkyl group of 1 to 4 carbon atoms or aryl;
Z is -H.HCl and R is -OR', where R' is a lineal alkyl group of 1 to 4 carbon atoms; and
Z is -CONH, and R is -OH.

The compounds most particularly preferred are the following:

(a) D-(-)-2-(2'hydroxyphenyl)glycine;
(b) Hydrochloric chloride of D-(-)-2-(2'-hydroxyphenyl)glycine;
(c) Potassium salt of D-(-)-N-1-(methoxycarbonilpropene-2-il)-α-amino-α-(2-hydroxyphenyl)acetic acid;
(d) Sodium salt of D-(-)-N-1-(methoxycarbonilpropene-2-il)-α-amino-α-(2-hydroxyphenyl)acetic acid;
(e) Methyl ester of hydrochloride of D-(-)-2-(2'-hydroxyphenyl)glycine; and
(f) D-(-)-2-(2'-hydroxyphenyl)glycinamide.

The compounds of formula (I) may be obtained by the general process shown in the following reaction scheme.

## REACTION SCHEME

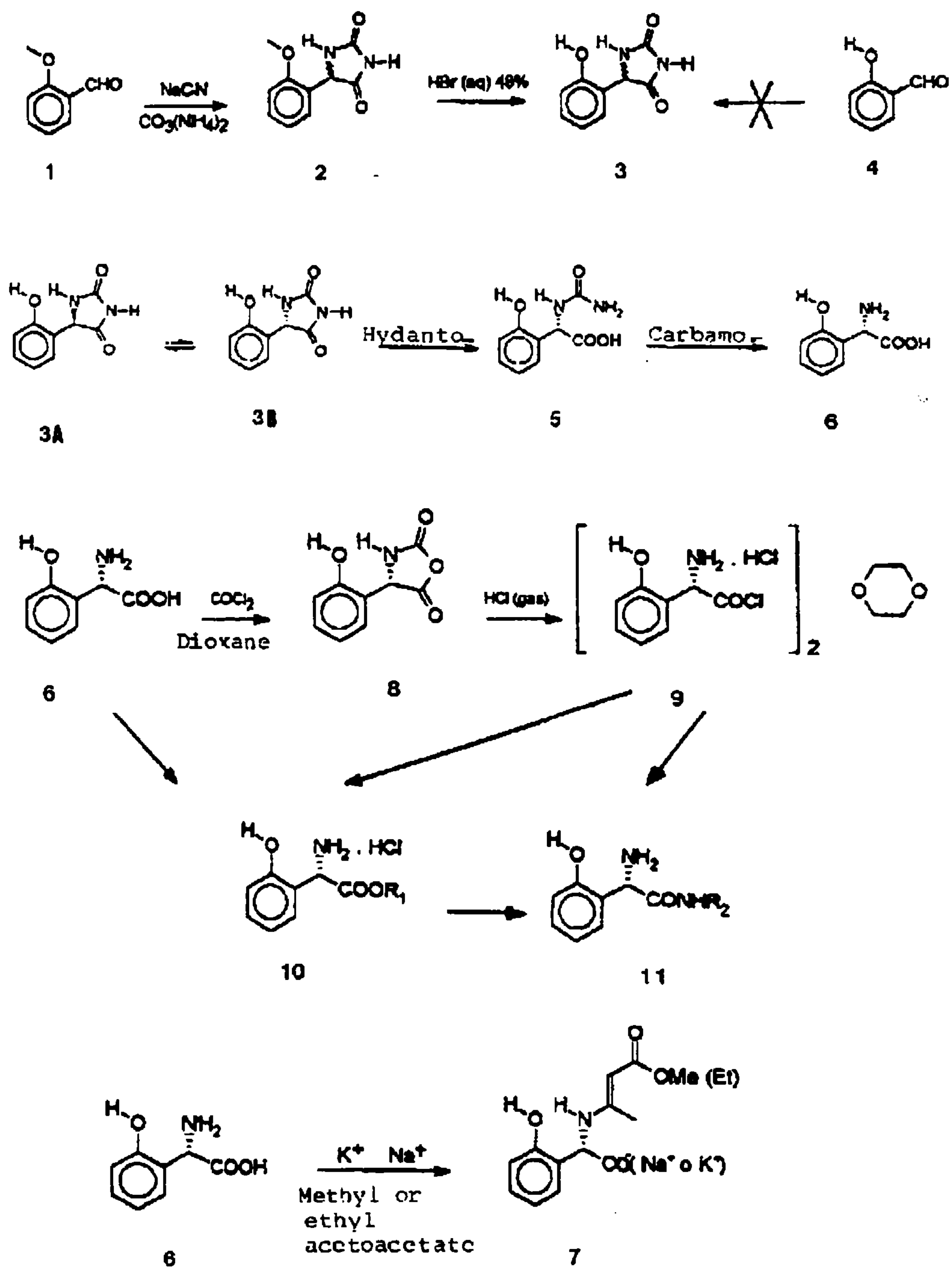


1 2 3 4

3A 3B 5 6

6 8 9

10 11

6 7

As may be seen, the direct synthesis of D, L,-5-(2'-hydroxyphenyl)-hydantoin (3) by the Bucherer-Berg reaction between salicylaldehyde (4), sodium cyanide and ammonium carbonate only results in polymerisation products. It is therefore necessary to use an indirect route in which the hydroxyl group of the aromatic ring is protected as a methyl ether [2-methoxybenzaldehyde (1)]. According to the process described by Henze and Speer [J. Am. Chem. Soc., 64, 523 (1942)], the reaction of 2-methoxybenzaldehide (1) with sodium cyanide and ammonium carbonate gives rise to the corresponding methoxylate derivate (2). Hydrolysis of the ether group of the compound (2) with 48% hydrobromic acid gives D,L-5-(2'-hydroxyphenyl)hydantoin (3) with a moderate yield.

For obtaining D-(-)-2-(2'-hydroxyphenyl)glycine (6) by an enzymatic method, it is possible to use the corresponding racemic hydantoin (3), this being hydrolysed with the D-(-)-amino acid by means of a biocatalyzer which contains enzymes having hydantoinate and carbamolase activity. The said biocatalyzer may be a microorganism which contains enzymes having hydantoinate and carbamolase activity, such as strains of different microorganisms, amongst which there are *Agrobacterium radiobacter* [Serge Runser, Nicolas Chinksi and Eric Ohleyer, Appl. Microbiol. Biotechnol. (1990) 33: 382-388], *Arthrobacter crystallopoietes* [A. Möller, C. Syldatk, M. Schulze and F. Wagner, Enzyme Microb. Technol. (1988), Vol. 10: 618] or *Pseudomonas sp* [K. Yokozeki, S. Nakamori, Ch. Eguchi, K. Yamada and K. Mitsugi, Agric. Biol. Chem. 51 (2), 355-362. (1987)]. For carrying out the process these microorganisms may be free or fixed using conventional techniques known to technicians in this field. Additionally, the biocatalyzer may be constituted of enzymatic extracts obtained by sound treatment or by the pure and fixed enzymes. The D,L-5-(2'-hydroxyphenyl) hydantoin (3) racemises spontaneously in the working conditions of biological systems, therefore attaining complete transformation to D(-)-amino acid, according to the sequence of reactions shown in the Reaction Scheme. Enzymatic reaction with entire cells is carried out in a phosphate buffer, at a pH controlled between 6.9 and 7.5, and at a working temperature between 35° C to 45° C, although preferentially between 39° C to 42° C. The concentration of the hydantoin loading may vary from 3% to 10%, while preferentially it should be at 3%.

As may be seen in the Reaction Scheme, the sodium or potassium salt of the acid D-(-)-N-1-(alcoxycarbonilpropene-2-il)-α-amino-α-(2'-hydroxyphenyl)acetic (7) may be obtained by reaction of the D-(-)-2-(2'-hydroxyphenyl) glycine (6) with sodium or potassium carbonate, or with sodium or potassium hydroxide and the methyl or ethyl ester of the acetoacetic acid in a water-alcohol medium. A low molecular weight alkanol may be used for the alcohols, such as methanol, ethanol, isopropanol, isobutanol and similar agents. If the base used is a carbonate, it is therefore convenient to screen the base as it is not soluble in an water-alcohol medium.

The obtaining of hydrochloric chloride of D-(-)-2-(2'-hydroxyphenyl)glycine (9) may be undertaken by means of reaction with phosgene in dioxane and the opening of cyclic anhydride (8) (Leuch's anhydride) with HCl gas, according to the method of Williams et al. [United States patent No. US 3,925,418 (1974)]. The hydrochloric chloride may be isolated as a hemisolvate with the dioxane. Shortly, for obtaining the compound (9), the amino acid (6) which has a particle size less than 200 micrometers (μm) is suspended in dioxane at a ratio of 1:10 (w:v) and phosgene is passed through during 10 minutes, until a total of 1.8 moles of phosgene per mole of amino acid has been reached. The reaction mixture is heated to 64° and once the reaction is finished, the remnants of phosgene are eliminated and the solution is vacuum concentrated. A mixture of toluene and dioxane is then added, and a current of hydrochloric acid is passed through during one hour. It is necessary to sow crystals of the product so that it crystallises.

Alternatively, the compound (9) may also be obtained through reaction with the adduct formed by the thionile chloride and the dimethylformamide (DMF) [R. Maggi et al., Italian patent No. IT 22323 A/79]. To this end, the previously prepared adduct is proportioned over the hydrochloride of amino acid (6) in dioxane at temperatures below 10° C. Once proportioning has ceased it is heated to a temperature of between 20° C and 30° C during 15 minutes. As was the case above, it is necessary to sow to obtain the finished product.

For the preparation of an ester of type (10) formula in which $R_1$, which is an alkyl group of 1 to 4 carbon atoms, it is possible to start on the direct basis of the chiral amino acid (6) by any conventional method, such as, for example, using thionyle chloride dissolved in the corresponding alcohol, or by reaction of the hydrochloride chloride (9) with the corresponding alcohol.

Finally, D-(-)-2-(2'-hydroxyphenyl)glycinamide (11) may be obtained by using chemical or enzymatic means. Using chemical means, this may be carried out by reaction of the methyl or ethyl ester corresponding to the amino acid (6) with an ammonia aqueous solution [Journal of Organic Chemistry, 52, 4379 (1987); Org. Syn. Coll., vol. 4, 516-536 (1955)], or by a mixture of ammonia and ammonium chloride [Org. Syn. Coll., vol 4, 486 (1963)]. When in the formula compound (11) $R_2$ is an alkyl group $C_1$-$C_4$, direct preparation may be undertaken using the treatment of hydrochloric chloride of D-(-)-2-(2'-hydroxyphenyl)glycine (9) with the corresponding amine.

D-(-)-2-(2'-hydroxyphenyl)glycinamide (11) may be obtained by using enzymatic means from racemic amide by reaction with a biocatalyzer with aminopeptidase activity. This said biocatalyzer may be composed of a microorganism, fixed or free, which contains L-aminopeptidase activity, such as a strain belonging to the species of *Pseudomonas putida* [Roos, E.C.; Mooiweer, H.H.; Heimstra, H.; Speckamp, W.N.; Kaptein, B.; Boesten, W.H.J.; Kamphuis, J.J., Org. Chem. (1992), 57, 6769], in which case, a suspension of fresh cells (recently fermented) of *Pseudomonas putida* with L-aminopeptidase (or L-amidase) activity may be added to a solution of the racemic amide in a phosphate buffer (0.1

M, pH 7.0). After 4 hours, the D-amide is separated from the L- amino acid by using benzaldehyde treatment. In another alternative embodiment for this process, the said biocatalyzer is composed of an enzymatic extract with L-aminopeptidase activity or a pure enzyme having this said activity [Meijer, E.M.; Boesten, W.H.J.; Schoemaker, H.E.; and van Balken, J.A.M., Biocatalysts in Organic Synthesis, Elsevier, Amsterdam, (1985), pages 135-156], preferentially fixed.

The alkaline and alkali earth metal salts of the compounds of formula (I) may be obtained by conventional methods known to experts in this field.

The invention provides new chiral derivates of hydroxyphenyl)glycine, such as D-(-)-2-(2'-hydroxyphenyl)glycine (6) and other chiral derivates of the D-(-)-amide, D-(-)-N-carbamoyl, D-(-)-ester and D-(-)-hydrochloride chloride type. These compounds of formula (I) may be used as intermediaries for the manufacture of a new range of β-lactamic antibiotics by chemical acylation using hydrochloric chloride or Dane's salt (enamines produced by reaction of sodium or potassium salts of D-(-)-2-(2'-hydroxyphenyl)glycine with the methyl or ethyl ester of acetoacetic acid) [Dane et al, Ang. Ch. Internat. Ed. 1, 658 (1962)], with the nuclei of 6-APA, 7-ACA and 7-ADCA or by enzymatic reactions of the amide or ester with the corresponding penicillin or cephalosporin rings. The R configuration of these compounds is preferred, as it is proven that when the configuration of the lateral chain in the antibiotic is R, it is biologically more active that the corresponding derivate with the S configuration.

The following examples illustrate the invention.

EXAMPLE 1

D.L-5-(2'-methoxyphenyl) hydantoin (2)

A mixture of 4 grams (0.0293 moles) of 2-methoxybenzaldehyde, 9.1 grams (0.948 moles) of ammonia carbonate and 2.6 grams (0.0399 moles) of potassium cyanide is suspended in 50 ml of a 50% mixture of methanol-water. The reactive mixture is kept at 50-60°C for three hours, resulting in complete dissolution. After eliminating the methanol by means of a rotary steam device, the residue is diluted with 50 ml of water, heated until re-dissolved, treated with active carbon and left to crystallize slowly. A yield of 85-90% above 2-methoxybenzaldehide was obtained. Melting point (189°C) is in accordance with the literature (Harvill and Herbst; cited above).

EXAMPLE 2

D.L-5-(2'-hydroxyphenyl) hydantoin (3)

6 grams of D, L-5-(2'-methoxyphenyl) hydantoin are suspended in 30 ml of hydrobromic acid at 48%. The suspension is placed in moderate reflux for 2-3 hours, which produces complete dissolution. The reaction is followed by means of high resolution liquid chromatography (HPLC) [HP column 125*4, flow 1.8 ml/min; λ = 212 nm; eluent: phosphate buffer / methanol (90/10), pH 2.5] until the methoxy-derivative disappears. After cooling and filtering, a 56% yield of a solid white cream is obtained with a melting point of 243°C (bibliography: 240-244°C with decomposition) [Harvill and Herbst; cited above].

IR (Tablet of KBr) λ = 3349 (f), 3252 (f), 3038 (f), 2762 (m), 1716 (f), 1596 (m), 1458 (m), 1429 (m), 1363 (m), 1194 (m), 748 (f) $cm^1$.

EXAMPLE 3

Obtaining D-(-)-2-(2'-hydroxyphenyl) glycine (6) by enzymatic means

D, L-5-(2'-hydroxyphenyl) hydantoin (3) is loaded in a 3% concentration into a phosphate buffer at pH 8. The biocatalyzer (whole *Agrobacterium radiobacter* cells) is loaded according to the following equation:

$$\frac{\text{grams of biological material - grams of Hydantoin * 100}}{\text{Ra*Rs}}$$

where Rs is the dry residue and Ra is the measurement of specific activity of the bio-mass (defined in this case as the ratio between grams of hydantoin and grams of dry residue which give a 65% conversion in 16 hours). The working temperature is 40°C. The working pH is maintained at 7.5 throughout the reaction.

By means of HPLC [HP column 125*4; Flow 1.8 ml/min; λ = 212 nm; eluent: phosphate buffer/methanol 90/10, pH 2.5] it is observed that the reaction ends at 48 hours.

The reaction mixture is acidulated at pH 0.5 with 98% sulphuric acid and the bio-mass is separated by filtering through decalite, or by centrifugation. Floating matter reaches a concentration of amino acid of approximately 20%. Next, it is neutralized to pH 4.3-4.5 with sodium hydroxide at 50%. The first precipitate appears, which is a mixture of amino acid and sulphate. After filtering, the mother waters are treated with methanol and they precipitate most of the salts. The methanol/water mixture is concentrated by rotary steam until dry. Both parts are then united and resuspended in hot pure methanol. After the last remains of salt have been eliminated, the methanol is filtered and dried. The yield over the initial hydantoin is 95%.

Optical rotation (c = 1, HC1 1N) = -158°
Melting point (uncorrected) = 193-194°C

The structure of the ortho-amino-acid is in accordance with the spectrographies carried out:

Mass spectrography (FAB technique) : molecular ion m/e 167.
$^1$H RMN (DMSO-d6) $\delta$ = 12 ppm (sa, COOH); 10 (sa, 1H, OH), 8.8 (sa 2H, $NH_2$), 6.7-7.2 (m, 4 H, aromatic hydrogens of the ortho system), 4.6 (s, CH).

The intermediate derived carbamoyl prepared by reaction of the amino-acid with potassic cyanate [T. Suzuki, K. Igarishi, K. Hase and K. Tuzimura, Agr. Biol. Chem., 37 (2), 411-416, (1973], is characterized by its spectrums:

Mass (FAB technique) : 421.2 ($2M^+$+1), 211.1 ($M^+$+1), 168.1; 154.1; 136.1; 79
1R (Nujol) $\lambda$ = 3950, 3340, 1675, 1543, 1450, 1375, 1290, 1110 $cm^{-7}$
$^{13}$C RMN $\delta$ = 173.51c; 158.57c; 155.107c; 129; 125.2c; 119.19; 115.69; 52.061.
$^1$H RMN $\delta$ = 12.4 ppm (sa, COOH); 9.8 (sa, OH); 6.7-7.2 (m, aromatic H); 6.5 (d, NH, J = 7.3 Hz); 5.7 (s, $NH_2$); 6.35 (d, CH, J = 7.3 Hz).

EXAMPLE 4

Potassium salt from the acid D-(-)N-1-(methoxycarbonylpropene-2-il)-$\alpha$-amino-$\alpha$-(2-hydroxyphenyl)acetic (DANORTO) (7)

200 ml of anhydric isopropanol (KF<0.5%) is placed within a 500 ml glass vessel and 20 g of D-(-)-2-(2'-hydroxyphenyl)glycine are suspended on it. Next, 7.76g of 99.5% filtered potassic carbonate (200 $\mu$m mesh), 14.4 ml of methyl aceto-acetate and 1 ml of 2-ethylhexanoic acid are loaded. The reaction is maintained at 70°C for 5 hours, resulting in complete dissolution. Residual cloudiness is filtered off through paper while warm. The transparent dissolution is cooled to 20°C and filtered. Finally it is washed with 100 ml of isopropanol. The product obtained is dried in a vacuum at 70°C. Dry weight: 30.8 g. Yield: 85%.

Optical rotation (c=4, water) = -87°

The spectrum of proton nuclear magnetic resonance is in accordance with the proposed structure.

$^1$H RMN (DMSO) $\delta$ = 9.35 (d, NH, J = 12 Hz); 6.6-7.1 (m, 4H, aromatic ring); 5 (d, OH, J = 12 Hz); 4.4 (s, =C-H); 3.5 (s, $OCH_3$); 1.55 (s, $CH_3$).

EXAMPLE 5

Hydrochloric chloride of D-(-)-2-(2'-hydroxyphenyl) glycine (9)

Following the procedure described by Williams et al., (North American patent No. US 3,925,418, cited above), hydrochloric chloride is obtained with a yield of 87% as hemisolvate with the dioxane. By means of potentiometric evaluation with methanol soda, the product has a richness greater than 95%.

Optical rotation (c = 1, methanol) = -117°

EXAMPLE 6

Methyl ester of hydrochloric D-(-)-2-(2'-hydroxyphenyl) glycine (10)

This titer compound was obtained from hydrochloric chloride (9). To this end, the hydrochloric chloride of D-(-)-2-(2'-hydoxyphenyl) glycine is re-suspended in chloroform (1:10 p.v.) and anhydrate methanol is proportioned slowly for two hours in a molar excess of 3 to 1. Once proportioning is finished, the reaction is maintained in reflux for a further two hours. At the end of this time, the excess alcohol is distilled. The product is then filtered and washed with chloroform. The yield over the initial hydrochloric chloride is 98%.

Optical rotation: (c = 1, methanol) = -125°.
$^1H$ RMN ($D_2O$) $\delta$ = 7.2-6.1 ppm (m, aromatic H); 4.72 (s, CH); 3.7 (s, $OCH_3$).
Mass spectrum (FAB technique) = molecular ion m/e 217.5.

EXAMPLE 7

D-(-)-2-(2'-hydroxyphenyl)glycinamide (11)

The methyl ester of hydrochloric D-(-)-2-(2'-hydoxyphenyl) glycine (10) is dissolved in 25% aqueous ammonia at 0°C. The ammonia must be in a molar excess of 3 to 1 over the ester which has been added. After two hours at this temperature, the product is extracted with ethyl acetate. The organic extract is dried over sodium sulphate and the solvent is evaporated in a vacuum. A yield of 70% of the amide, devoid of free amino acid, is obtained over the ester which was added.

optical rotation (c = 1, HC1 1N) = -127°.
$^1H$ RMN (DMSO) $\delta$ = 9.8 ppm (sa, OH); 7.5 (s, $CONH_2$); 7 (s, $NH_2$); 6.7-7.1 (m, aromatics H); 4.6 (s, CH).

**Claims**

1. Chiral derivates of hydroxyphenylglycine, with the D configuration, and having the general formula (I)

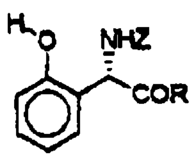

(I)

where

R is hydrogen, hydroxy, chloride, $-NH_2$, -NHW, where W is an alkyl group $C_1$-$C_4$, or aryl, -OR', where R' is an alkyl group $C_1$-$C_4$; and
Z is hydrogen, $CONH_2$ and -C ($CH_3$)=CHCOOY, where Y is methyl or ethyl;

their salts, solvates and hemisolvates.

2. A compound according to claim 1, in which Z is hydrogen and R is -OH.

3. A compound according to claim 1, in which Z is -C (CH3)=CHCOOY, where Y is methyl or ethyl and R is $-O^-K^+$ or $O^-Na^+$.

4. A compound according to claim 1, in which Z is - H.HCl and R is -CL, in the form of hemisolvate with dioxane.

5. A compound according to claim 1, in which Z is H and R is $-NH_2$ or NHW, and where W is a lineal alkyl group of 1 to 4 carbon or aryl atoms.

6. A compound according to claim 1, in which Z is - H.HCl and R is OR', and where R' is a lineal alkyl group of 1 to 4

carbon atoms.

**7.** A compound according to claim 1, in which Z is - $CONH_2$ and R is -OH.

**8.** A compound according to claim 1, selected from the group formed of:

(a) D-(-)-2-(2'hydroxyphenyl)glycine;
(b) Hydrochloric chloride of D -(-)-2-(2'-hydroxyphenyl)glycine;
(c) Potassium salt of acid D-(-)-N-1-(methoxycarbonilpropene-2-il)-$\alpha$-amino-$\alpha$-(2-hydroxyphenyl)acetic;
(d) Sodium salt of acid D-(-)-N-1-(methoxycarbonilpropene-2-il)-$\alpha$-amino-$\alpha$-(2-hydroxyphenyl)acetic;
(e) Methyl ester of hydrochloric D -(-)-2-(2'-hydroxyphenyl)glycine; and
(f) D -(-)-2-(2'-hydroxyphenyl)glycinamide.

**9.** An enzymatic procedure for obtaining D -(-)-2-(2'-hydroxyphenyl)glycine, according to claim 2, which includes the enzymatic hydrolysis of the corresponding racemic hydantoin with a biocatalyzer containing enzymes having hydantoinate and carbamoylase activity.

**10.** A procedure according to claim 8, in which the said biocatalyzer includes a microorganism, fixed or free, which contains enzymes having hydantoinate and carbamoylase activity.

**11.** A procedure according to claim 10, in which the said microorganism is selected from the group formed by strains of *Agrobacterium radiobacter, Arthrobacter crystallopoietes* and *Pseudomonas sp*.

**12.** A procedure according to claim 8, in which the said biocatalyzer includes a an enzymatic extract having hydantoinate and carbamoylase activity.

**13.** A procedure according to claim 8, in which the said biocatalyzer includes a pure enzymes having fixed hydantoinate and carbamoylase activity.

**14.** A procedure for obtaining sodium or potassium salts of the D-(-)-N-1-(alcoxycarbonilpropene-2-il)-$\alpha$-amino-$\alpha$-(2-hydroxyphenyl)acetic acid, that includes making the sodium or potassium salt of the D -(-)-2-(2'-hydroxyphenyl)glycine react with an ester of the acetoacetic acid in a water - alcohol mix.

**15.** A procedure for the preparation of an ester of hydrochloric D-(-)-2-(2'-hydroxyphenyl)glycine, which includes making D-(-)-2-(2'-hydroxyphenyl)glycine react with phosgene and hydrochloric acid gas in anhydric dioxane.

**16.** A procedure for the preparation of an ester of hydrochloric D-(-)-2-(2'-hydroxyphenyl)glycine, which includes making the hydrochloric chloride of D-(-)-2-(2'-hydroxyphenyl)glycine react with the corresponding anhydric alcohol.

**17.** A procedure for obtaining D-(-)-2-(2'-hydroxyphenyl)glycinamide, which includes making an ester of hydrochloric D-(-)-2-(2'-hydroxyphenyl)glycine react with aqueous ammonia.

**18.** An enzymatic procedure for the preparation of D-(-)-2-(2'-hydroxyphenyl)glycinamide, which includes making the corresponding racemic acid react with a biocatalyzer that contains L-aminopeptidase activity.

**19.** A procedure according to claim 18, in which the said biocatalyzer is composed of a microorganism, free or fixed, which contains L-aminopeptidase activity.

**20.** A procedure according to claim 19, in which the said microorganism is a strain belonging to the *Pseudomono putida* species.

**21.** A procedure according to claim 19, in which the said biocatalyzer is composed of an enzymatic extract which presents L-aminopeptidase activity.

**22.** A procedure according to claim 19, in which the said biocatalyzer is composed of a pure enzyme which has fixed L-aminopeptidase activity.

## INTERNATIONAL SEARCH REPORT

International application No.
PCT/ES 96/00226

A. CLASSIFICATION OF SUBJECT MATTER

Int. Cl.[6] C07C229/36, 237/20, C12P13/04, 41/00

According to International Patent Classification (IPC) or to both national classification and IPC

B. FIELDS SEARCHED

Minimum documentation searched (classification system followed by classification symbols)

Int. Cl.[6] C07C C12P

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P, X | Chem. Abs. (Columbus, Ohio, U.S.A.) abstract 126 : 104418, F. SATOH et al. : "Preparation of 2-hydroxy-phenylalkylamine derivatives as Maillard" RN=185339-08-6 : Benzeneacetic acid,.alpha. -amino-2-hydroxy-, (5)- See abstract and WO 9636591 A (KISSEI PHARM.) 21.11.96 | 1,2 |
| X | ES 2060187 A (GYOGYSZER) 16.11.94 see claims 2, 6-8, 10-14 | 1,3,8,14 |
| X | PATENT ABSTRACTS OF JAPAN, Vol. 12. n° 450 (C-547), 1988 and JP 63173595 A (MITSUI) 18.07.88 see abstract | 8,9 |
| A | US 4094741 A (YAMADA) 13.06.78 see examples 1,5 - 14 | 1,2,8 |
| A | EP 614882 A (WESTSPUR) 14.09.94 | 1-21 |
| P,A | WO 9525715 (CIBAGEIGY) 28.09.95 | 1-21 |

[X] Further documents are listed in the continuation of Box C. [X] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 February 1997 (25.02.97) | 4 March 1997 (04.03.97) |
| Name and mailing address of the ISA/ S.P.T.O. | Authorized officer |
| Facsimile No. | Telephone No. |

Form PCT/ISA/210 (second sheet) (July 1992)